# EUROPEAN PATENT APPLICATION

(11) **EP 4 691 404 A1**
(43) Date of publication of application: **11.02.2026**
(21) Application number: 24306326.0
(22) Date of filing: 06.08.2024
(51) Int. Cl.: A61B 34/20, A61B 90/00, A61B 90/30

(54) **METHOD FOR OBTAINING A 3D INTRAOPERATIVE IMAGE WITH A DEPTH CAMERA**

(71) Applicant: Kyniska Robotics, 38400 Saint-Martin-d'Hères (FR)
(72) Inventor: DAANEN, Vincent, 38400 SAINT MARTIN D'HERES (FR)
(74) Representative: Regimbeau

(57) **Abstract**

The invention concerns a computer implemented method, comprising the steps of:
- positioning, at several positions around a region of interest (ROI) of a patient (1), an assembly (3) comprising a depth camera (4) rigidly attached to a registration device (5), the registration device (5) being disposed in the field of view of the depth camera (4), the registration device (5) comprising a registration device tracker (9);
- acquiring, by means of the depth camera (4), for each position of a plurality of positions around the region of interest, a set of points comprising a first cloud of 3D points representing the region of interest and a second cloud of 3D points representing the registration device;
- locating, for each position, the registration device tracker (9) relatively to a patient tracker (8) attached to the patient (1) by means of a locating system (7);
- obtaining, for each position, from the location of the registration device tracker (9) relatively to the patient tracker of a transformation ^{™}T_{PM} from a coordinate system of the registration device tracker (9) to a coordinate system of the patient tracker;
- determining a transformation ^{C}T_{MP} from a coordinate system of the depth camera to a coordinate system of the patient, the transformation being computed by the following equation ^{C}T_{PM}= ^{C}T_{TP} ^{TP}T_{TM} ^{™}T_{PM} with C a coordinate system attached to the depth camera, PM a coordinate system attached to the patient tracker, TM a coordinate system attached to the registration device tracker (9), TP a coordinate system attached to the registration device (5);
- processing, from the transformation ^{C}T_{MP} at each position, each set of points in order to obtain, in the coordinate system attached to the patient tracker, a corresponding cloud of 3D points of the region of interest (ROI);
- combining each cloud of 3D points of interest in the coordinate system attached to the patient tracker for obtaining a resulting cloud of 3D points of the region of interest representing the region of interest.

## Description

### FIELD OF THE INVENTION

The invention relates to the field of computer assisted surgery and in particular to bone tracking in such surgery.

### BACKGROUND OF THE INVENTION

Computer assisted surgery (CAS) is a medical technology using computer systems to assist surgeons in planning, guiding and executing surgical procedures with high precision and efficiency.

During a surgery, a dense geometric representation of a part of interest of the patient anatomy is needed to assist the surgeon. Usually, this representation is based on preoperative images obtained by CT scans or MRI scans from which a 3D geometric representation of a part of interest of the patient anatomy is obtained. Then, a precise registration between the geometric data extracted from the preoperative images and the intraoperative data acquired during the surgery is required. The intraoperative data are often obtained either by palpation or by manually moving a hand-held camera and are thus long and complex to obtain. Also, intraoperative acquisitions of CT or MRI scans during the surgery require special operating rooms (equipped with aforementioned scanners). It is however possible to use imaging devices such as CBCT or (3D) ultrasound scanners and extract a 3D geometric representation of the part of interest of the patient anatomy from the data but this way to acquire the patient anatomy obviously requires additional means (scanners), processing and intraoperative checking (to validate the results of the processing).

### BRIEF DESCRIPTION OF THE INVENTION

The invention permits to obtain a 3D intraoperative representation of a region of interest of a patient anatomy during a surgery.

According to a first aspect, the invention concerns computer implemented method, comprising the steps of:
- positioning, at several positions around a region of interest of a patient, an assembly comprising a depth camera rigidly attached to a registration device, the registration device being disposed in the field of view of the depth camera, the registration device comprising a registration device tracker;
- acquiring, by means of the depth camera, for each position of a plurality of positions around the region of interest, a set of points comprising a first cloud of 3D points representing the region of interest and a second cloud of 3D points representing the registration device;
- locating, for each position, the registration device tracker relatively to a patient tracker attached to the patient by means of a locating system;
- obtaining, for each position, from the location of the registration device tracker relatively to the patient tracker of a transformation ^{™}T_{PM} from a coordinate system of the registration device tracker to a coordinate system of the patient tracker;
- determining a transformation ^{C}T_{MP} from a coordinate system of the depth camera to a coordinate system of the patient, the transformation being computed by the following equation ^{C}T_{PM}= ^{C}T_{TP} ^{TP}T_{TM} ^{TM}T_{PM} with C a coordinate system attached to the depth camera, PM a coordinate system attached to the patient tracker, TM a coordinate system attached to the registration device tracker, TP a coordinate system attached to the registration device;
- processing, from the transformation ^{C}T_{MP} at each position, each set of points in order to obtain, in the coordinate system attached to the patient tracker, a corresponding cloud of 3D points of the region of interest;
- combining each cloud of 3D points of interest in the coordinate system attached to the patient tracker for obtaining a resulting cloud of 3D points of the region of interest representing the region of interest.

The invention according to the first aspect comprises the following features alone or in combination:
- the step of processing comprises filtering each set of points to select the points belonging to a bone.
- the filtering takes into account the position of the second cloud of 3D points representing the registration device for identifying whether the points belong to the bone or the registration device, the points belonging to the registration device being in the same area in the field of view of the depth camera.
- the filtering is based on the color and/or texture of the points for identifying whether the points belong to the bone or the registration device.
- the filtering consists in a selection of the bone manually in an image representing the set of points.
   - the registration device comprises a support plate on which, on a first face, characteristics elements are disposed, the first face being if front of the depth camera, the
   characteristics elements allowing orientation of the set of points in relation to each other, the characteristics elements raising from the first face.
- the registration device comprises at least three characteristics elements, at least two characteristics elements having different volumes.
- the characteristics elements have identical geometrical shapes.
- the characteristics elements have different geometrical shapes.
- the characteristics elements comprise three cylinders.
- the depth camera is rigidly attached to the registration device by means on a longitudinal arm having a length between 20 cm and 50 cm.
- it comprises a step of displaying images representing each set of point and/or the resulting cloud of 3D points on a display unit.
- the region of interest is a knee of a patient the patient tracker being attached to the tibia and/or the femur of the patient, the locating step, the transformation ^{™}T_{PM} and the transformation ^{C}T_{MP} being obtained for each patient tracker, each image being processed in relation to each patient tracker.

According to a second aspect, the invention concerns a device comprising:
- a depth camera configured for acquiring cloud of 3D points of a region of interest;
- a registration device tracker rigidly attached to the depth camera, the registration device comprising a support plate on which, on a first face, characteristics elements are disposed, the first face being in front of the depth camera so that the depth camera is able to acquire a first cloud of 3D points of the region of interest along with a second cloud of 3D points of the registration device;
- a localization system configured to locate the registration device relatively to a patient tracker;
- a processing unit configured to implement a method according to the first aspect of the invention.

According to a third aspect, the invention concerns a computer program product comprising instructions which, when the program is executed by a computer, cause the computer to carry out the steps of the method according to the first aspect of the invention.

### BRIEF DESCRIPTION OF THE DRAWINGS

Further features and advantages of the invention will be apparent from the description that follows, based on the appended drawings, wherein:
- Figure 1 illustrates a surgical scene according to an embodiment;
- Figure 2 illustrates a top view of a registration device according to an embodiment;
- Figure 3 illustrates a side view of a registration device according to an embodiment;
- Figure 4 illustrates a method for obtaining a 3D intraoperative representation of a region of interest of a patient according to an embodiment;
- Figure 5 illustrates the surgical scene with several positions of a depth camera around a patient with a corresponding representation of the cloud of 3D points acquired with the depth camera and a resulting cloud of 3D points obtained with the method for obtaining a 3D intraoperative representation of a region of interest of a patient according to an embodiment.

On the figures, similar elements have identical references.

### DETAILED DESCRIPTION OF EMBODIMENTS OF THE INVENTION

The described method relates to a computer-implemented method for obtaining a 3D intraoperative representation of a region of interest of a patient and to obtain a resulting cloud of 3D points of this region. The aim of the method is to combine accurately several cloud of 3D points acquired by means of a depth camera in several positions around the patient for obtaining a resulting cloud of 3D points combining each cloud of 3D points.

### Presentation of the surgical scene and of the representing assembly

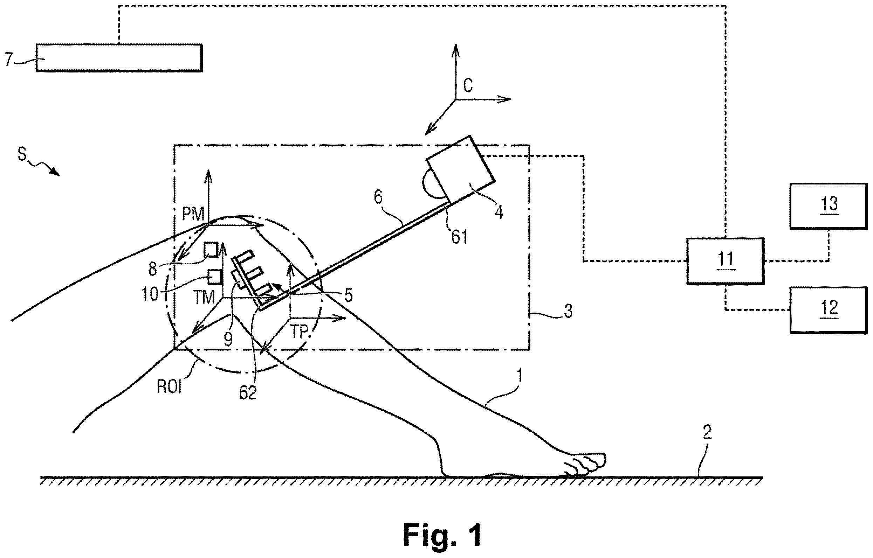

Figure 1 shows a surgical scene S for the knee surgery wherein a leg 1 lying on a table 2. A representing assembly 3 is disposed on a surgical scene S for intraoperative representing the knee.

The representing assembly 3 comprises a depth camera 4 attached to a registration device 5. The depth camera 4 is preferably attached to a robotic surgical arm (not shown) for displacing the depth camera 4 around the area to be imaged. A robotic surgical arm is for instance described in document EP4279010A1. It can be understood that the invention is not limited to surgery but applies to any field wherein a robotic arm is involved.

The depth camera 4 is configured to acquire 3D representation in a form of cloud(s) of points of elements that are disposed in the field of view of the camera. Each point of the cloud represents a space coordinate.

The depth camera 4 is disposed at a first end 61 of an arm 6 and the registration device 5 is disposed at a second end 62 of the arm 6. The arm 6 is made of a rigid material. Its length shall accommodate the working range of the camera. A good range for the length is 30cm to 50cm.

The material and finishing of the arm shall be compatible with the localization system. By example, high reflective material shall be avoided in the case of optical localization system; and metallic material shall be avoided in the case of electromagnetic localization system. Using a depth camera allows to acquire the external geometry of an object/area of interest as cloud of 3D points. Some depth cameras also provide other geometric information such as normal and/or curvature which might be key information in some cases (e.g., surface reconstruction).

When coupled with a RBG sensor, the camera (in this case, the cameras designated by "RGB+D camera") also provides the color information for every point of the returned point of the cloud.

A localization system 7 permits to locate both the registration device 5 and a patient tracker 8 and aims at locating a registration device tracker 9 relatively to the patient tracker 8. For instance, for the surgery of the knee, the femur or tibia can be located. On figure 1, a patient tracker 8 is rigidly attached to the femur and the registration device tracker 9 is rigidly attached to the registration device 5.

The localization system 7 can be optical or electromagnetic. In case of an electromagnetic localization system the registration device tracker 9 and the patient tracker 8 are each an electromagnetic receiver and an electromagnetic transmitter 10 is disposed in the surgical field. By processing the transmitted and received signals, the localization system 7 permits to locate the registration device tracker 9 relatively to the patient tracker 8. In case of an optical localization system, the registration device tracker 9 and the patient tracker 8 are adapted to be detected by the localization system 7.

The depth camera 4 and the localization system 7 are connected to a processing unit 11 configured to implement a method for obtaining a 3D representation of a region interest of a patient as it will be described.

A storage unit 12 and a display unit 13 are connected to the processing unit 11 for storing data acquired and for displaying the various representations during the method.

### Registration device 5

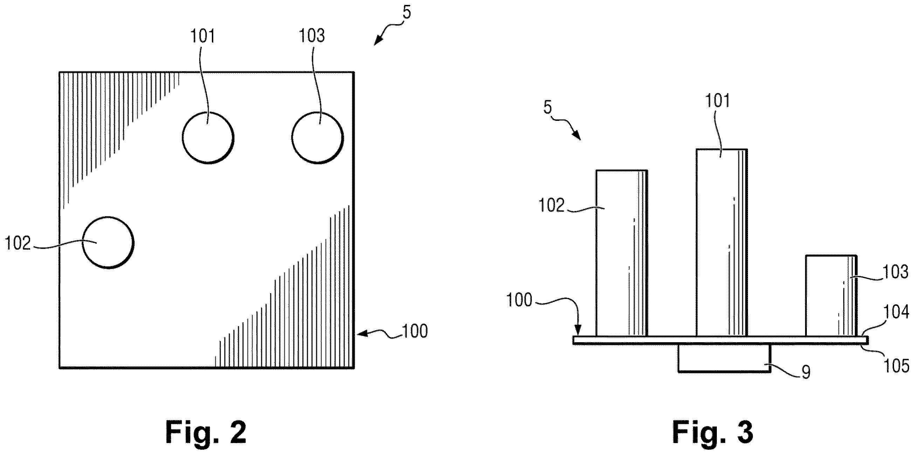

Figure 2 shows a top view of the registration device 5 and figure 3 shows a side view of the registration device 5 according to an embodiment.

The registration device 5 comprises a support plate 100, preferably a rectangular plate, on which characteristics elements 101, 102, 103 are arranged. These characteristics elements 101, 102, 103 can be of any kind as long as 1) they are correctly visible by the depth camera 4 and 2) they allow to determine the correct orientation of the registration device 5 in its corresponding 3D representation obtained with the depth camera. The characteristics elements are for instance cylinders of different volumes (height, radius). Of course, other geometric shapes are possibles. The registration device 5 comprises at least three characteristics elements, preferably four characteristics elements.

According to an embodiment the registration device 5 comprises at least three characteristics elements 101, 102, 103, at least two characteristics elements having different volumes with same or different geometrical shapes. As already presented, the registration device 5 is rigidly attached to the depth camera 4 so that when the depth 4 camera is moving, the registration device 5 is already at the same position relatively to the depth camera 4 and in the same area in the field of view of the depth camera 4.

The registration device 5 comprises a registration device tracker 9 configured to locate the registration device 5. The registration device tracker 9 is disposed on the support plate 100. For instance, the characteristics elements 101, 102, 103 are disposed on a first side 104 of the support plate 100 while the registration device tracker 9 is disposed on a second side 105 of the support plate 100 opposed to the first side 104. The location of the registration device tracker 9 in relation to the registration device 5 is known likewise the location of the depth camera 4 in relation to the registration device 5 and so to the registration device tracker9.

### Method

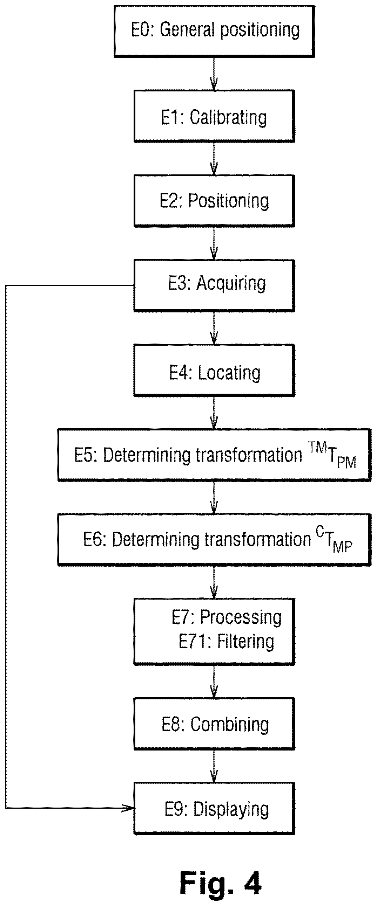

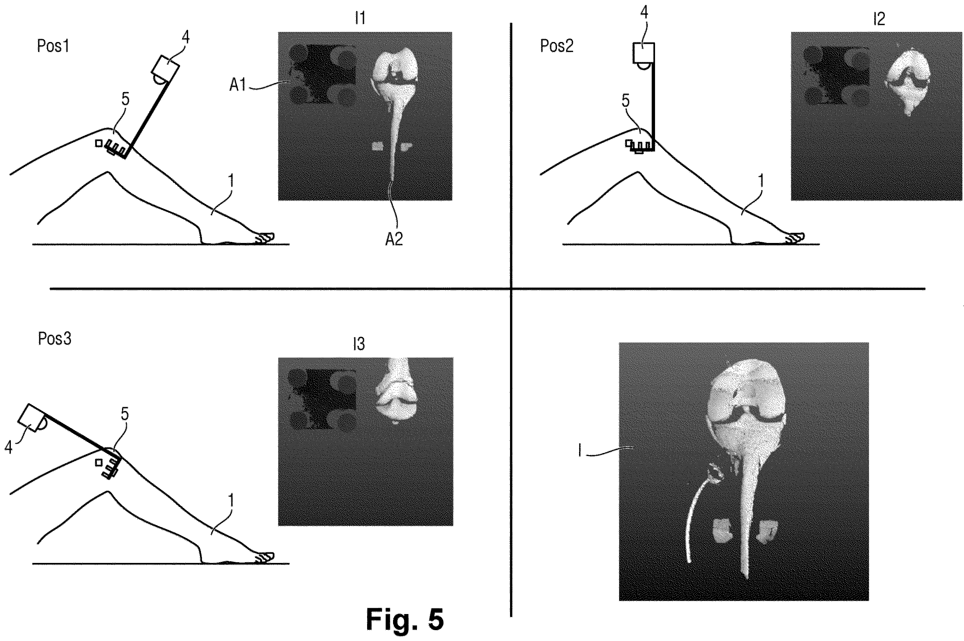

A method for obtaining a 3D representation of a region interest of a patient is described in reference to figure 4 and figure 5.

A representing assembly 3 is positioned in the surgical scene at the vicinity of the patient by means preferably of a robotic arm (step E0) after the surgery started. In case of the surgery of the knee, an incision has been made and the bones are visible. In particular, the depth camera 4 is positioned over a region of interest (the knee here) with the registration device 5 attached to the depth camera 4 so that the registration device is disposed in the field of view of the depth camera.

Once the representing assembly 3 is positioned in the surgical scene, a calibrating step (step E1) is implemented for locating the registration device 5 in relation to the depth camera 4 and in relation to the registration device tracker 9. This step can be repeated during the process if needed for ensuring a correct localization between the depth camera 4, the registration device tracker 9 and the registration device 5. This calibrating step permits to locate a coordinate system TP attached to the registration device 5 in relation to a coordinate system C attached to the depth camera 4.

The representing assembly 3 is then positioned at several positions around the region of interest of a patient (step E2). Three positions Pos1, Pos2, Pos2 can be seen on figure 5.

For each position, a set of points representing the region of interest ROI and the registration device 5 is acquired by the depth camera 4 (step E3). In particular, a first cloud of 3D points concerns the region of interest and a second cloud of 3D points concerns the registration device 5.

Figure 5 illustrates three images I1, I2, I3 representing each set of points acquired by the depth camera 4 for each position Pos1, Pos2, Pos3. On these images I1, I2, I3, the region of interest is visible on a first area A1 of the image and the registration device 5 is visible in a second area A2 of the image. Since the camera is a depth camera the images represent cloud of 3D points, each point having 3D coordinates in the coordinate system C of the depth camera 4. It can be noticed that for each set of points acquired the second cloud of 3D points of the registration device 5 is always in the same position in relation to the second cloud of 3D points allowing representations to be oriented in relation to one another with the coordinate system C of the depth camera and the coordinate system TP of the registration device 5.

Thus, for each position of the representing assembly in particular, the depth camera 4 and the registration device 5:
- a first cloud of 3D points representing the region of interest and a second cloud of 3D points representing the registration device 5 are obtained;
- an image I1, I2, I3 wherein in a first area the first cloud of 3D points representing the region of interest is visible and in a second area a second cloud of 3D points representing the registration device tracker is visible, can be obtained;
- a location of the registration device tracker 9 in relation to the patient tracker 8 can be obtained.

This latter location permits to obtain (step E5), for each position, a transformation ^{™}T_{PM} from a coordinate system of the registration device tracker to a coordinate system of the patient tracker.

The method permits to combine the set of points obtained from several positions for obtaining a 3D representation of the region of interest ROI in a form of a cloud of 3D points.

To combine them, it is necessary to have them in a same coordinate system in particular the one attached to the patient and more particular the one attached to the patient tracker MP.

For each position a transformation ^{C}T_{MP} from a coordinate system of the depth camera 4 to a coordinate system of the patient is computed (step E6). This transformation being computed by the following equation ^{C}T_{PM}= ^{C}T_{TP} ^{TP}T_{TM} ^{TM}T_{PM} with C the coordinate system attached to the depth camera, PM a coordinate system attached to the patient tracker, TM a coordinate system attached to the registration device tracker, TP the coordinate system attached to the registration device.

^{C}T_{TP} and ^{TP}T_{TM} are known by construction and ^{TM}T_{PM} is determined by localizing the registration device tracker 9 in relation to the patient tracker 8 during the locating step (step E5). Additionally, ^{C}T_{TP} can be refined during the calibration steps to correct for small changes (due to gravity, material wear, etc.).

Once the transformation ^{C}T_{PM} is computed, each set of points is processed (step E7) in order to transform them in the coordinate system attached to the patient tracker.

According to an embodiment the step of processing (step E7) comprises filtering (step E71) each set of points to select the points belonging to a bone. The filtering permits to only select the points corresponding to the bones.

The filtering can be made taking into account the position of the cloud of 3D points representing the registration device for identifying whether the points belong to the bone or the registration device, the points belonging to the registration device being in the same area in the field of view of the depth camera.

The filtering can be based on the color and/or texture of the points for identifying whether the points belong to the bone or the registration device tacker.

According to an embodiment, the filtering (step E71) consists in a selection of the bone manually in the representation of the set of points acquired using a display of each image.

The processed set of points (eventually filtered) are then combined together (step E8) for obtaining a resulting cloud of 3D points of the region of interest ROI representing the region of interest. This resulting representation comprises the contribution of each acquired set of points. The resulting cloud of 3D points can be displayed as image I as seen on figure 5.

Preferably, each set of points and/or the resulting cloud of 3D points can be displayed on a display unit 13.

## Claims

1. Computer implemented method, comprising the steps of:
- positioning (E2), at several positions around a region of interest (ROI) of a patient (1), an assembly (3) comprising a depth camera (4) rigidly attached to a registration device (5), the registration device (5) being disposed in the field of view of the depth camera (4), the registration device (5) comprising a registration device tracker (9);
- acquiring (E3), by means of the depth camera (4), for each position of a plurality of positions around the region of interest, a set of points comprising a first cloud of 3D points representing the region of interest and a second cloud of 3D points representing the registration device;
- locating (E4), for each position, the registration device tracker (9) relatively to a patient tracker (8) attached to the patient (1) by means of a locating system (7);
- obtaining (E5), for each position, from the location of the registration device tracker (9) relatively to the patient tracker of a transformation ^{™}T_{PM} from a coordinate system of the registration device tracker (9) to a coordinate system of the patient tracker;
- determining (E6) a transformation ^{C}T_{MP} from a coordinate system of the depth camera to a coordinate system of the patient, the transformation being computed by the following equation ^{C}T_{PM}= ^{C}T_{TP} ^{TP}T_{TM} ^{TM}T_{PM} with C a coordinate system attached to the depth camera, PM a coordinate system attached to the patient tracker, TM a coordinate system attached to the registration device tracker (9), TP a coordinate system attached to the registration device (5);
- processing (E7), from the transformation ^{C}T_{MP} at each position, each set of points in order to obtain, in the coordinate system attached to the patient tracker, a corresponding cloud of 3D points of the region of interest (ROI);
- combining (E8) each cloud of 3D points of interest in the coordinate system attached to the patient tracker for obtaining a resulting cloud of 3D points of the region of interest representing the region of interest.

2. Method as claimed in claim 1, wherein the step of processing (E7) comprises filtering (E71) each set of points to select the points belonging to a bone.

3. Method as claimed in claim 2, wherein the filtering (E71) takes into account the position of the second cloud of 3D points representing the registration device (5) for identifying whether the points belong to the bone or the registration device, the points belonging to the registration device being in the same area in the field of view of the depth camera.

4. Method as claimed in claim 2, wherein the filtering (E71) is based on the color and/or texture of the points for identifying whether the points belong to the bone or the registration device.

5. Method as claimed in claim 2, wherein the filtering (E71) consists in a selection of the bone manually in an image representing the set of points.

6. Method as claimed in claims 1 to 5, wherein, the registration device (5) comprises a support plate (100) on which, on a first face (104), characteristics elements (101, 102, 103) are disposed, the first face (104) being if front of the depth camera (4), the characteristics elements (101, 102, 103) allowing orientation of the set of points in relation to each other, the characteristics elements raising from the first face (104).

7. Method as claimed in claims 1 to 6, comprising at least three characteristics elements (101, 102, 103), at least two characteristics elements having different volumes.

8. Method as claimed in claim 7, wherein the characteristics elements (101, 102, 103) have identical geometrical shapes.

9. Method as claimed in claim 7, wherein the characteristics elements (101, 102, 103) have different geometrical shapes.

10. Method as claimed in claims 7 to 9, wherein the characteristics elements (101, 102, 103) comprise three cylinders.

11. Method as claimed in claims 1 to 10, wherein the depth camera (4) is rigidly attached to the registration device (5) by means on a longitudinal arm (6) having a length between 20 cm and 50 cm.

12. Method as claimed in claims 1 to 11, comprising a step of displaying (E9) images (I1, I2, I3) representing each set of point and/or the resulting cloud of 3D points (I) on a display unit.

13. Method as claimed in claims 1 to 12, wherein the region of interest is a knee of a patient the patient tracker (8) being attached to the tibia and/or the femur of the patient, the locating step (E4), the transformation ^{™}T_{PM} and the transformation ^{C}T_{MP} being obtained for each patient tracker, each image being processed in relation to each patient tracker.

14. A device comprising:
- a depth camera (4) configured for acquiring cloud of 3D points of a region of interest;
- a registration device (5) tracker rigidly attached to the depth camera (4), the registration device comprising a support plate on which, on a first face, characteristics elements are disposed, the first face being in front of the depth camera (4) so that the depth camera (4) is able to acquire a first cloud of 3D points of the region of interest (ROI) along with a second cloud of 3D points of the registration device;
- a localization system (7) configured to locate the registration device relatively to a patient tracker;
- a processing unit (11) configured to implement a method according to claims 1 to 13.

15. A computer program product comprising instructions which, when the program is executed by a computer, cause the computer to carry out the steps of the method of any one of claims 1 to 13.
